(19) Europäisches Patentamt
European Patent Office
Office européen des brevets

(11) **EP 4 185 199 B1**

(12) **EUROPEAN PATENT SPECIFICATION**

(45) Date of publication and mention
of the grant of the patent:
**26.03.2025 Bulletin 2025/13**

(21) Application number: **21743586.6**

(22) Date of filing: **08.07.2021**

(51) International Patent Classification (IPC):
*A61B 5/08* (2006.01)   *A61B 5/09* (2006.01)
*A61B 5/097* (2006.01)   *A61B 5/087* (2006.01)
*F01D 5/12* (2006.01)   *G01F 1/10* (2006.01)

(52) Cooperative Patent Classification (CPC):
**A61B 5/09; A61B 5/0873; A61B 5/097; G01F 1/10;**
A61B 2560/0406; A61B 2560/0462;
A61B 2562/0233; F01D 5/12

(86) International application number:
**PCT/IB2021/056140**

(87) International publication number:
**WO 2022/018555 (27.01.2022 Gazette 2022/04)**

(54) **IMPROVED TURBINE FOR SPIROMETER**

VERBESSERTE TURBINE FÜR SPIROMETER

TURBINE AMÉLIORÉE POUR SPIROMÈTRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.07.2020 IT 202000017818**

(43) Date of publication of application:
**31.05.2023 Bulletin 2023/22**

(73) Proprietor: **MIR - MEDICAL INTERNATIONAL
RESEARCH S.P.A.**
**00173 Roma (IT)**

(72) Inventor: **BOSCHETTI SACCO, Paolo**
**00135 Roma (IT)**

(74) Representative: **Ripamonti, Enrico et al
Giambrocono & C. S.p.A.,
Via Rosolino Pilo, 19/B
20129 Milano (IT)**

(56) References cited:
**EP-A1- 0 369 506        EP-A2- 1 967 694
CN-U- 210 494 075        JP-A- 2000 298 043
JP-A- 2000 298 044        US-A- 3 989 406
US-A1- 2020 129 091**

**Description**

FIELD

The subject of the present disclosure is a turbine for a spirometer.

BACKGROUND

As is known, a spirometer is a medical instrument which is used to measure the volumes and flows of pulmonary air, and the respiratory functionality of a patient.

An important element of the spirometer is constituted by the measurer for the flow and volume of air which, amongst the various types which are suitable for carrying out this function, can be a removable turbine (including of the "disposable" type), used for respiratory analysis during inhalation and exhalation. The turbine usually comprises a main tubular body, which can be transparent, a rotor with small blades facing one another, which is placed centrally relative to this body, and rotates around a longitudinal axis of the tubular body, and two deflectors, which are usually identical, disposed at, or in the vicinity of, an intake opening and an output opening of the tubular body. The rotor is thus interposed between the deflectors.

The turbine is associated with a seat provided in a body of the spirometer.

By means of infrared sensors which are integral with this body of the spirometer, there is detection in a known manner of the rotation of the rotor (or mobile equipment of the spirometer), and calculation of the flow of air entering from the "intake" deflector (i.e. which deflector is placed at the intake opening of the tubular body).

Often, a nozzle is also associated with this opening.

By means of known methods, the pulmonary functionality of the patient is detected from the flow of air entering.

Each deflector usually comprises an annular body which is inserted in the tubular body of the turbine, and is blocked there for interference; this annular body is integral with a plurality of blades which are secured on a fulcrum placed centrally relative to the annular body, and disposed along a longitudinal axis of the deflector (i.e. of its annular body), coinciding with the longitudinal axis of the tubular body of the turbine.

The blades define a plurality of sectors (or openings) through which the air inhaled or exhaled by the patient passes during a spirometric measurement.

The deflectors retain the rotor between them, and permit the rotation thereof under the action of the flow of air generated (inhaled or exhaled) by the patient.

Two factors are very important in a spirometer, that is, the resistance to the flow of air or loss of load through the turbine, and the threshold of the minimum measurable air flow (which then leads to stoppage of the rotor).

In particular, the resistance to the air flow is quantified as the mean pressure difference between the intake of the air into the turbine (or into the nozzle which may be associated therewith), and the external air, i.e. the ambient pressure.

As far as the minimum flow threshold is concerned, this is the flow value that determines the stoppage of the rotation of the rotor, which is a fundamental parameter for evaluating the capacity of the rotor to continue its own movement when the air flow admitted into the turbine is equal to a predefined lower limit (the minimum flow threshold).

These values are standardised by standards published by pneumological associations and by ISO (International Organisation for Standardisation) international standards which are compulsory for all producers of spirometers. In particular, according to the standards, the maximum resistance must be less than 1.5 cm $H_2O$/L/s throughout the entire measurement interval of the flow; for the minimum flow threshold this is defined by standards which require the flow measurer of the spirometer (in this case the turbine) to be able to measure a minimum flow (the "threshold") equal to 25 ml/s.

In the definition of the aforementioned factors, the dimensional characteristics of the components of the turbine are quite important; in particular, the characteristics which affect the performance levels of a turbine directly are:

- number of sectors of the deflector and gap between them;
- curvature of the surface;
- geometry of the channels: dimensions of the diameter at the intake (deflector) correlated to the central diameter at the rotor;
- geometry of the fulcrum;
- dimensions of the rotor.

In addition, an important part in the definition of the aforementioned factors is played by the structure of the blades of the deflector.

Prior art documents EP0369506A1, US2020/129091A1 and CN210494075U, all disclose details of the design of turbines, rotor and deflectors for spirometers.

SUMMARY

The objective of the present invention is to provide a turbine for a according to appended claim 1 which makes it possible to obtain the best performance levels by the medical instrument with which it is associated.

The objective of the invention is to provide a turbine of the aforementioned type which makes it possible to maximise the directing of the flow of air onto the rotor, consequently maximising the component of the thrust force generated by this flow on the rotor.

Another objective is to provide a turbine of the aforementioned type in which the effect of the air on the rotor is optimised in minimum measurable flow conditions.

A further objective is to provide a turbine of the aforementioned type which is simple to produce and has restricted costs, such as to permit disposable use (or single use) thereof.

A further objective is to provide a turbine of the aforementioned type which is not affected by the environmental conditions of temperature, humidity and pressure, and which is in conformity with the stringent accuracy standards prescribed by the pneumological associations in the sector, i.e. ERS/ATS.

Another objective is to provide a turbine with a deflector which guarantees that the minimum measurable flow threshold is obtained ($< 25$ mL/s), and which at the same time does not involve any increase, but if possible reduction, of the resistance to the flow (the ERS and ATS standards prescribe that this resistance should be $< 1.5$ cm $H_2O$/L/s).

These objectives and others, which will become apparent to persons skilled in the art, are achieved by a turbine according to the appended claims.

BRIEF DESCRIPTION OF THE DRAWINGS

For better understanding of the present invention, and purely by way of non-limiting example, the following drawings are appended, in which:

figure 1 shows a spirometer in perspective view provided with a turbine according to the invention;
figure 2 shows a perspective view of the turbine of figure 1 associated with a nozzle;

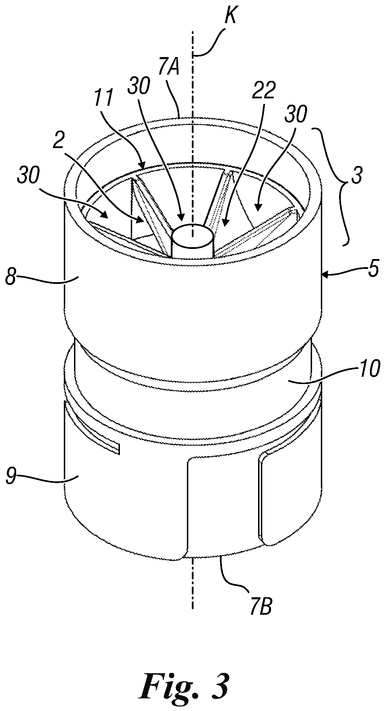

figure 3 shows a perspective view of the turbine;

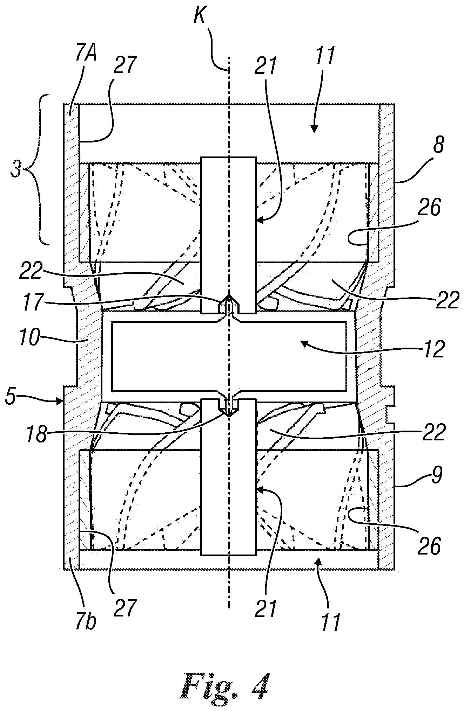

figure 4 shows a view in longitudinal cross-section of the turbine;

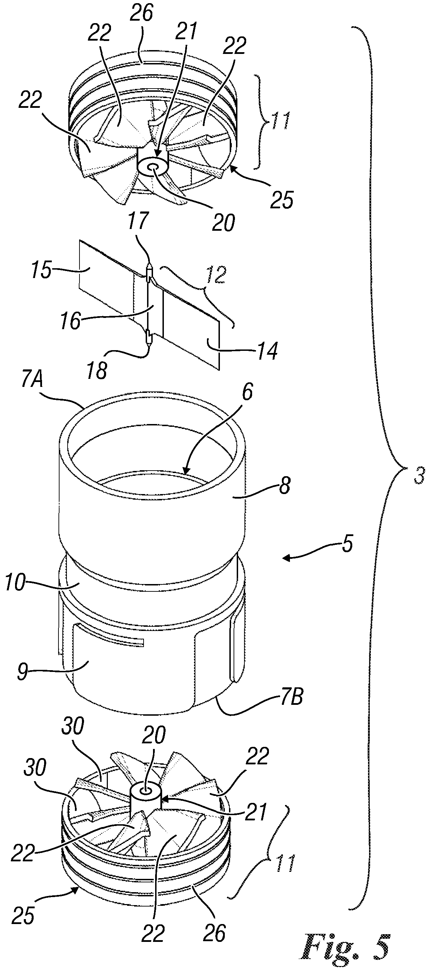

figure 5 shows an exploded perspective view of the turbine;
figure 6 shows a view of the curve which is representative of the development of a blade of the turbine (or external generatrix curve) at the outermost part thereof, i.e. the external development of the blade at the annular body of the deflector projected on a plane;
figure 7 shows a view of the curve which is representative of the development of the blade of the turbine of figure 6 at the fulcrum of the deflector (or internal generatrix curve), i.e. projected onto this fulcrum;
figure 8 shows a front view of the blades of a deflector of the turbine according to the invention;
figure 9 shows a perspective view of a blade of the deflector of figure 8; and
figures 10 and 11 show the development of the flows of air between the two deflectors of the turbine.

DETAILED DESCRIPTION

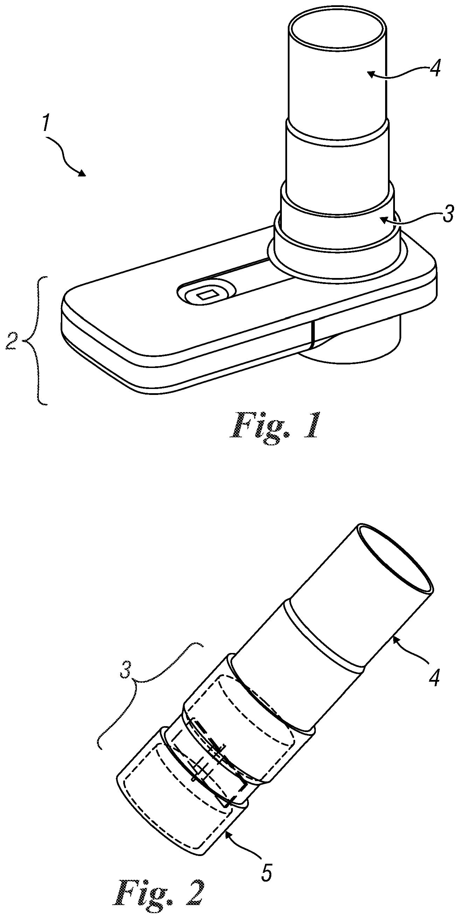

With reference to the aforementioned figures, figure 1 shows a spirometer 1 with a body 2, with which a turbine 3 is associated, in any known manner and detachably. A nozzle 4 is connected to the turbine by coupling by means of interference. The turbine 3 comprises a main body 5 which can be transparent. The body 5 is substantially tubular, and has a through-hole 6. This tubular body 5 comprises an opening 7A for intake of the air, and an opening 7B for output of the air, and corresponding intake 8 and output 9 portions. Between these, a central portion 10 is present. The definitions of intake and output of the turbine are interchangeable, since in the exhalation phase the air travels in one direction, whereas in the inhalation phase, for obvious reasons, the air travels in the opposite direction.

In the intake and output portions 8 and 9, there are placed corresponding deflectors 11, which have a form identical to one another, whereas in the central portion 10, there is present a conventional rotor 12 which can rotate around a longitudinal axis K of the tubular body 5. For this purpose, this rotor has a known form with small blades 14 and 15 facing one another, and projecting orthogonally from a hub 16 which has projections 17 and 18 facing one another, disposed along the axis K and able to rotate in seats 20 of corresponding fulcrums 21 of the deflectors 11.

Each of these deflectors, which are inserted by interference in the respective intake or output portions 8, 9 of the tubular body 5, comprises blades 22 which project from said fulcrum 21, which is placed along a longitudinal axis of the deflector 11 coinciding with the longitudinal axis K of the tubular body 5. The blades end at, and are fixed to a cylindrical annular body 25 of the deflector (with a wall 26 lying parallel to the axis K), which body can cooperate in conveying the flow directly together with the internal wall 27 of the corresponding intake or output portion 8, 9 of the tubular body 5.

The blades 22 are identical to one another, and have the same form; this is in order to avoid irregularities in the flow of air between sectors 30 which are present between said blades (the "gaps" present between the blades), with consequent irregular thrust of the air on the rotor, which thrust, on the contrary, as far as possible, must remain homogenous at any given moment of use of the turbine.

From the point of view of circulation of the air through the deflectors 11, each of the deflectors has a passive surface and an active surface.

"Active surface" means the surface of each blade on which the air flows freely towards the rotor 12 or from the rotor 12.

"Passive surface" on the other hand means the surface which opposes the intake of the air into the deflector; in practice this surface is equal to the sum of all the surfaces which do not contribute to the movement of the air within the sectors 30, and which thus do not contribute to the rotation of the air which passes into the deflector, and which ensures the movement of the rotor 12 (and, in the final analysis, operation of the turbine and correct use of the spirometer 1). This passive surface is therefore represented by the thickness of end intake portions 35 (with reference to the flow of air) of the blades 22 and by the thickness of the body 25.

Consequently, the active surface of the deflector is provided by the sum of all the surfaces of the blades 22, placed between a corresponding end intake edge or portion 35, and an end output edge or portion 36 of said blades, which, when supplied with the air, generate rotary movement thereof which contributes to the rotation of the rotor 12. It should be noted that the end edges or portions 35 of the blades 22 are in the interior of the annular body 25 of the deflector 11, whereas the end output portions 36 project from this body.

It is therefore of fundamental importance to have correct design of the active surface of each blade, i.e. of the surface which is presented by each blade to the flow of air, or which is supplied with the flow of air, and directs the air input into the turbine.

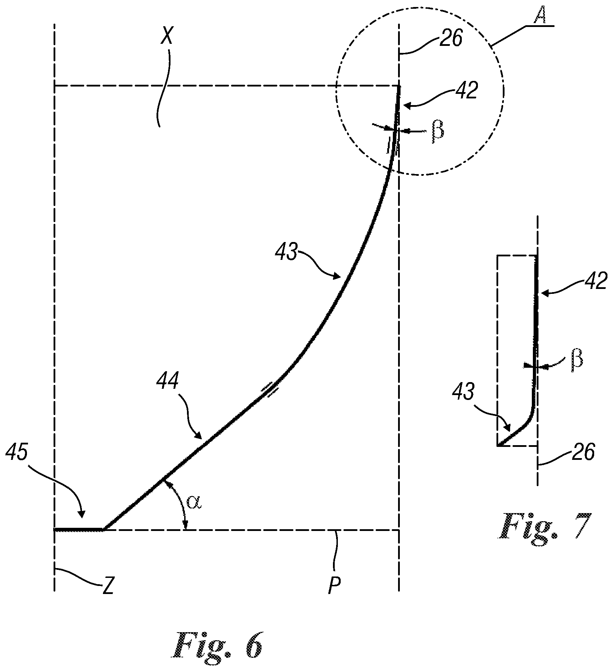

In this respect, the active surface of each blade which is supplied with the air inhaled or exhaled by a patient is defined by a succession of adjacent, consecutive parts with a spatial form which is variable going from the end intake portion 35 to the end output portion 36; "spatial form which is variable" means that the parts which define the active surface have different geometric characteristics; they can be flat or curved surfaces (as will be described hereinafter), which, on any plane X orthogonal to a plane Z which contains the axis K (see fig. 6, where the plane 7 is orthogonal to the drawing sheet), form a line which converges towards this plane Z. The flow of air laps the surface of the blade and follows the configuration thereof, thus according to a path corresponding to the curve shown in figure 6.

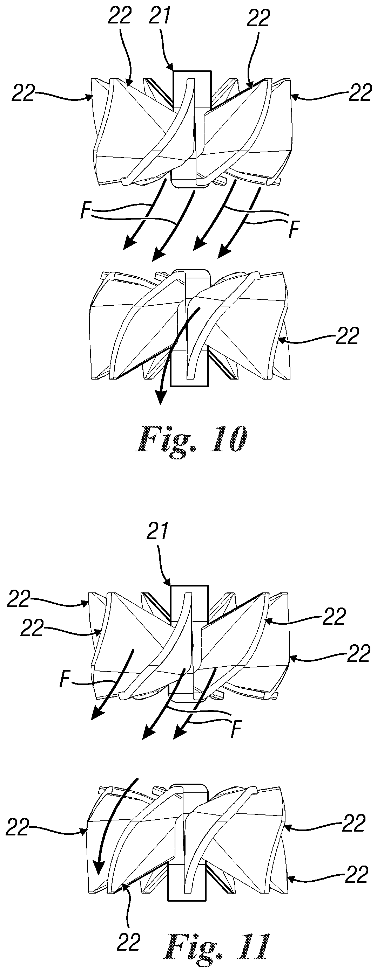

More particularly, starting from the end intake edge or portion 35 (see figures 6, 7 and 9, 10), each blade comprises a first "section of surface" 42 (i.e. a first part of its surface) which is flat and straight in cross-section as schematised in figure 6, but is inclined with respect to the (cylindrical) wall 26 of the annular body 25 (having generatrices which lie spatially parallel to the longitudinal axis K of the tubular body 5).

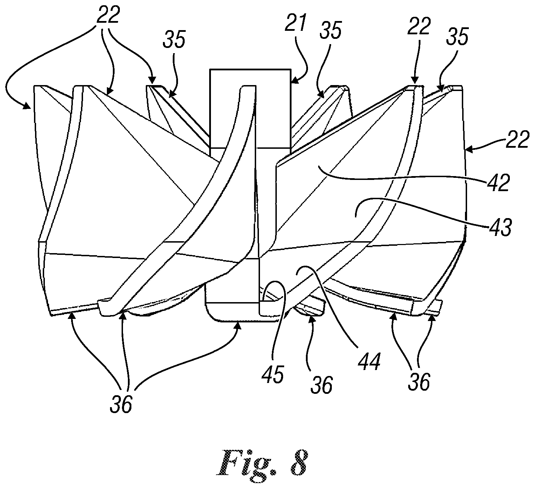

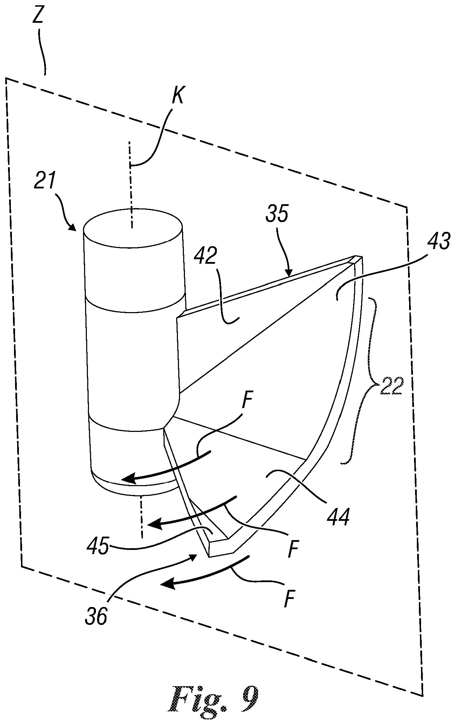

From the first section of surface 42 (see figures 8 and 9) there extends a second section or part of surface of the blade 43 which is curved (i.e. defined by a curved surface) ending in a third section or part of surface of the blade 44 which is also flat (i.e. defined by a flat surface), but more inclined than the wall 26 of the annular body 25 with respect to the first section of blade 42. The third section or part of surface ends in a fourth section or part of surface of the blade 45 which, according to the present invention, is also flat (i.e. defined by a flat surface), straight in all of its transverse cross-section (as schematised substantially in figure 6), lies on a plane orthogonal to the axis K, and has an arrangement such as to deflect the flow of air which passes onto it in a direction which is orthogonal to the plane Z containing the longitudinal axis K of the tubular body 5 (as well as the axis of rotation of the rotor 12). By this means, when a small blade 14 and 15 of the rotor is at the fourth section 45 of a blade 22 of the deflector 11, and air is passing along the blade, this small blade receives a thrust from the air which leaves the blade 22 in a direction orthogonal to the small blade of the rotor 14 or 15. This fourth section, which is known as the "miniskirt" serves the purpose of maximising the thrust on the rotor of the flow of air in transit, since this flow is orthogonal to the surface of the small blade.

It should be noted that the fourth section 45 is orthogonal to the plane Z (see fig. 6), with the term "orthogonal" meaning an arrangement of the fourth section or part 45 on a plane which forms an angle of 90° with said plane Z, which forms an angle substantially equal to 90° (i.e. at the most equal to 90° ±5°) taking into consideration the normal construction tolerances associated with production of the deflector 11 by means of moulding of plastic material.

The fourth section or miniskirt 45 preferably has limited extension along the end output portion 36 of the rotor blade 22, and ends along this portion 36 without reaching the fulcrum 21, in order not to create excessive resistance to the passage of the flow of air inside the deflector.

The development of the fourth section of the surface of the blade thus has a direct effect on the minimum measurable flow threshold: all the fourth sections 45 of the blades 22 give rise to more efficient directing of the flow onto the rotor, according to a component which is normal to the small blades 14 and 15 of the rotor 1, which flow is responsible for the thrust on the small blade 14 or 15 itself; this therefore results in a greater thrust on the rotor for the same air load moving in the deflector, compared with a solution in which the blades of the deflector have an active surface without an "end" section in the vicinity of the end output portion 36 orthogonal to the plane Z containing the axis of the deflector K. It will be appreciated that the effect tends to be reduced until it is exhausted when the minimum measurable flow threshold is approached.

The use of the fourth section or "miniskirt" 45 at the end output portion 36 of the deflectors has made it possible to obtain a significant advantage relating to the performance levels of the turbine 3. In particular, it has been observed that, for the same resistance provided against the passage of the flow of air by each blade, an increase is obtained in the normal component of the speed of the flow orthogonally to the rotor; this results in an improved thrust for the same energy expenditure.

Thus, the miniskirt affects the air in transit in the turbine 3, creating for the same energy expenditure a normal component of the tangential speed of this air which maximises the thrust in the direction which is incident orthogonally on each small blade 14 or 15 of the rotor. This gives rise to reduction of the minimum measurable flow threshold, which is one of the two fundamental objectives for a turbine intended for measurement of spirometry. The result is an increase in the performance levels which, for each value of the load, are directly proportional to the maximum tangential speed possible on the mean plane of the rotor.

The fourth section or miniskirt 45 extends along a part of the output portion 36 of the blade, from the annular body 25 towards the fulcrum 21, so as not to give rise to a significant increase in the resistance to the flow of air, while making it possible to increase the tangential speed of the flow itself.

The effect of improvement derived from this configuration of the fourth section or miniskirt represents an innovative element which overcomes a technical disadvantage according to which a plane which is placed perpendicularly to the flow of air passing in the deflector from the intake portion 35 to the output portion 36 prevalently results in energy expenditure. The solution according to the invention, relating to the fact that the plane orthogonal to the flow does not pass along the entire length of the edge of the output portion of the deflector, but only along a part of this portion 36, has made it possible on the contrary to obtain optimum directing of the flow, thus avoiding the additional energy expenditure.

The section or miniskirt 45 is thus orthogonal (to within the small tolerances of $\pm 5°$ determined by the construction methods of the deflector) to the plane Z containing the longitudinal axis K of the deflector, so as to deflect the flow of air orthogonally to this axis K and to the blades 14 and 15.

As stated, each blade 22 has a succession of consecutive, adjacent parts or sections.

More particularly, the first section of surface 42 can form an angle $\beta$ of between 0.3° and 1.2°, preferably and advantageously between 0.5° and 0.7°, with the wall 26 of the annular body schematised in figure 7. The flat section of surface (or third, straight section) 44 forms an angle $\alpha$ with a plane P (orthogonal to the planes Z and X) which contains the fourth section 45, of between 37° and 41°, preferably of between 39° and 40° (which corresponds to an angle substantially of between 53° and 49° and 50° and 51° respectively between the extension of this section 44 and the wall 27 of the annular body 25).

Thanks to the form of each blade 22 (represented by the generatrix curves in figures 6 and 7), there is efficient intake (or output) of the air into/from each deflector 11. In fact, starting from the end intake portion 35 and ending with the output portion 36, each blade is defined by a succession of sections or parts of surface which are connected to one another with continuity, and which channel and accelerate appropriately the air input into each sector 30 of the deflector 11: the first section of surface or flat section 42 has the function of channelling the air which is accelerated thanks to the combination between said first section of surface and the second curved section of surface 43. The remaining sections of surface 44 and 45 make the air flow by directing it directly towards the rotor (substantially following the development of the curve 6 and flowing onto each blade 22).

It should be noted that the final section of surface or fourth section of blade 45 serves the purpose of maximising the thrust of the flow of air in transit towards the rotor 12 by directing the flow of air orthogonally relative to the small blade, which at a given moment is present at the relative blade 22 of the deflector.

Thanks to the form of each blade 22, a direct effect is obtained on the reduction of the minimum measurable flow threshold: the various sections or parts of blade 42-45 give rise to directing of the flow of air onto the rotor which is more efficient than that which can be obtained with the solutions of the prior art, consisting of blades with a surface with a continuous curvature without a final section lying on a plane orthogonal to that which contains the longitudinal axis of the deflector (or of the fulcrum 21 thereof); this form maximises the normal component which is responsible for the thrust on the rotor. This results in a greater thrust for the same load.

In the conditions of minimum flow of air input, use of the fourth section of surface of the blade 45 makes it possible to exert an efficient thrust on the rotor 12, with the result of lowering the minimum measurable flow threshold (as indicated by the arrows F in figures 9, 10 and 11).

It should be noted that the development of the surface has a direct effect on the minimum measurable flow threshold: the surfaces give rise to more efficient directing of the flow onto the rotor, thus maximising the normal component responsible for the thrust. This therefore results in a greater thrust for the same load. The effect tends to be reduced until it is exhausted when the minimum measurable flow threshold is approached.

The overall surface of each individual blade 22 can be described by means of a mathematical equation, while being subdivided into four sections of surface, three of which are flat, and one of which is curved.

In particular:

a) the first section of surface 42, the second section 43 and the third section 44 can be generalised by means of a non-linear function of the third order of the coordinate z as a function of the coordinates x and y:

$$f(x,y) = cost+ax+by+cx^2+dy^2+exy+fx^3+gy^3+hx^2+y+ixy^2$$

The parameters which describe this formula in the reference system of the device according to the figure are:

| cost | 21.83 |
|------|-------|
| a | -1.239 |
| b | 4.225 |
| c | -0.0396 |
| d | 0.224 |
| e | -0.032 |
| f | 0.001 |
| g | 0.0019 |
| h | -0.0069 |
| i | 0.064 |

b) the fourth section of surface 45 is defined by a linear function of the first order,

$$f(x,y) = cost+ax+by$$

wherein:

| cost | 76.679 |
|------|--------|
| a | 0.959 |
| b | 8.737 |

It will be appreciated that the data given in the tables are preferable from amongst those which allow the four surfaces to be developed and interconnected, but these data are given by way of indication. Small differences from these values are acceptable.

A preferred form of the invention has been described. Others are however possible, and come within the scope of the invention as defined by the following claims.

**Claims**

1. Turbine (3) for a spirometer (1) comprising a hollow tubular body (5) containing a rotor (12) which is mobile around a longitudinal axis (K) of this tubular body (5) and two deflectors (11) placed at an intake opening (7A) and at an output opening (7B) of said body (5), the rotor (12) comprising small blades (14, 15) which are facing one another and disposed between said deflectors (11), the deflectors comprising an annular body (25) and a fulcrum (21) disposed along a longitudinal axis of this annular body (25) coinciding with the longitudinal axis (K) of the tubular body (5), between said fulcrum (21) and said annular body (25) a plurality of blades (22) being provided which are integral with the fulcrum itself and with the annular body, said blades (22) delimiting a plurality of sectors (30) for the passage of air directed towards the rotor, and having an end portion for intake of the air (35) and an end portion for output of the air (36), each blade (22) having a continuous surface development which is defined by a plurality of adjacent, consecutive sections of surface which have a curved form and a flat form, and are interconnected to one another, **characterised in that**, at the end portion for output of the air (36), each blade (22) has an end section of surface (45) which is flat, straight in all of its transverse cross-section, said flat section being on a plane (P) perpendicular to the longitudinal axis (K) of said tubular body (5) and orthogonal to a plane (Z) containing said longitudinal axis (K), said flat end section of surface protruding towards the adjacent blade, said end section of surface (45) being configured to direct the air output from the deflector (11) orthogonally to said plane (Z) containing the longitudinal axis (K) of the tubular body and orthogonally onto the rotor (12).

2. Turbine according to claim 1, **characterised in that** the adjacent, consecutive sections of each blade (22) comprise a single section defined by a curved surface (3).

3. Turbine according to claim 1, **characterised in that** there are four (42-45) adjacent, consecutive sections of surface of each blade (22).

4. Turbine according to claim 3, **characterised in that**, starting from the input end portion (35) of the blade (22) and as far as the output end portion (36) of the blade (22), the blade comprises a first section of surface (42) defined by a flat surface, a second section of surface (43) defined by a curved surface, a third section of surface (44) defined by a flat surface, and a fourth section of surface defining the end section of surface (45) lying on a plane, the end section of surface (45) being inclined with respect to the third section of surface (44).

5. Turbine according to claim 4, **characterised in that** the first section of surface (42) forms an angle ($\beta$) with a wall (26) of the annular body (25) of between 0.3° and 0.9°, preferably of between 0.5° and 0.7°.

6. Turbine according to claim 4, **characterised in that** the third section of surface (44) forms an angle ($\alpha$) with respect to the plane (P) which contains the end section of surface (45) of between 37° and 41°, preferably of between 39° and 40°.

7. Turbine according to claim 1, **characterised in that** the end section of surface has a limited extension along the end output portion (36) of the air of each blade (22).

8. Turbine according to claim 4, **characterised in that**:

   a) the first section of surface (42), the second section of surface (43) and the third section of surface (44) can be defined by means of a non-linear function of the third order of the coordinate z as a function of the coordinates x and y:

   $$f(x,y) = cost + ax + by + cx^2 + dy^2 + exy + fx^3 + gy^3 + hx^2y + ixy^2$$

   b) the end section of surface (45) is defined by a linear function of the first order,

   $$f(x,y) = cost + ax + by$$

**Patentansprüche**

1. Turbine (3) für ein Spirometer (1), umfassend einen hohlen röhrenförmigen Körper (5), der einen Rotor (12), der um eine Längsachse (K) dieses röhrenförmigen Körpers (5) beweglich ist, und zwei Deflektoren (11), die an einer Einlassöffnung (7A) und an einer Auslassöffnung (7B) des Körpers (5) angeordnet sind, enthält, der Rotor (12) umfassend kleine Schaufeln (14, 15), die einander gegenüberliegen und zwischen den Deflektoren (11) angeordnet sind, die Deflektoren umfassend einen ringförmigen Körper (25) und einen Drehpunkt (21), der entlang einer Längsachse dieses ringförmigen Körpers (25) angeordnet ist, die mit der Längsachse (K) des rohrförmigen Körpers (5) zusammenfällt, wobei zwischen dem Drehpunkt (21) und dem ringförmigen Körper (25) eine Vielzahl von Schaufeln (22) bereitgestellt ist, die mit dem Drehpunkt an sich und mit dem ringförmigen Körper fest verbunden sind, wobei die Schaufeln (22) eine Vielzahl von Sektoren (30) für den Durchgang von auf den Rotor gerichteter Luft begrenzen und einen Endabschnitt zum Einlassen der Luft (35) und einen Endabschnitt zum Auslassen der Luft (36) aufweisen, wobei jede Schaufel (22) eine kontinuierliche Flächenentwicklung aufweist, die durch eine Vielzahl von angrenzenden, aufeinanderfolgenden Flächenabschnitten definiert ist, die eine gekrümmte Form und eine flache Form aufweisen und miteinander verbunden sind, **dadurch gekennzeichnet, dass** jede Schaufel (22) an dem Endabschnitt zum Ausgeben der Luft (36) einen Endabschnitt mit einer Fläche (45) aufweist, die in ihrem gesamten Querschnitt flach, gerade ist, wobei der flache Abschnitt in einer Ebene (P) senkrecht zu der Längsachse (K) des rohrförmigen Körpers (5) und orthogonal zu einer Ebene (Z) ist, die die Längsachse (K) enthält, der flache Endabschnitt der Fläche in Richtung der angrenzenden Schaufel hervorsteht, wobei der Endabschnitt der Fläche (45) konfiguriert ist, um den Luftaustritt aus dem Deflektor (11) orthogonal zu der Ebene (Z), die die Längsachse (K) des rohrförmigen Körpers enthält, und orthogonal auf den Rotor (12) zu richten.

2. Turbine nach Anspruch 1, **dadurch gekennzeichnet, dass** die angrenzenden, aufeinanderfolgenden Abschnitte jeder Schaufel (22) einen einzigen Abschnitt umfassen, der durch eine gekrümmte Fläche (3) definiert ist.

3. Turbine nach Anspruch 1, **dadurch gekennzeichnet, dass** es vier (42-45) angrenzende, aufeinanderfolgende Flächenabschnitte von jeder Schaufel (22) gibt.

4. Turbine nach Anspruch 3, **dadurch gekennzeichnet, dass** die Schaufel ausgehend vom Eingangsendabschnitt (35) der Schaufel (22) bis zu dem Ausgangsendabschnitt (36) der Schaufel (22) einen ersten Flächenabschnitt (42), der durch eine flache Fläche definiert ist, und einen zweiten Flächenabschnitt (43), der durch eine gekrümmte Fläche definiert ist, einen dritten Flächenabschnitt (44), der durch eine flache Fläche definiert ist, und einen vierten Flächenabschnitt, der den auf einer Ebene liegenden Endabschnitt von Fläche (45) definiert, umfasst, wobei der Endabschnitt der Fläche (45) in Bezug auf den dritten Abschnitt der Fläche (44) geneigt ist.

5. Turbine nach Anspruch 4, **dadurch gekennzeichnet, dass** der erste Abschnitt von Fläche (42) einen Winkel (β) mit einer Wand (26) des ringförmigen Körpers (25) zwischen 0,3° und 0,9°, vorzugsweise zwischen 0,5° und 0,7°, bildet.

6. Turbine nach Anspruch 4, **dadurch gekennzeichnet, dass** der dritte Flächenabschnitt (44) einen Winkel (α) mit der Ebene (P), die den Endflächenabschnitt (45) enthält, zwischen 37° und 41°, vorzugsweise zwischen 39° und 40°, bildet.

7. Turbine nach Anspruch 1, **dadurch gekennzeichnet, dass** der Endabschnitt der Fläche eine begrenzte Erstreckung entlang des Endausgangsabschnitts (36) der Luft jeder Schaufel (22) aufweist.

8. Turbine nach Anspruch 4, **dadurch gekennzeichnet, dass**:

a) der erste Flächenabschnitt (42), der zweite Flächenabschnitt (43) und der dritte Flächenabschnitt (44) mittels einer nichtlinearen Funktion dritter Ordnung der Koordinate z abhängig von den Koordinaten x und y definiert sein können:

$$f(x,y) = \text{cost}+ax+by+cx^2+dy^2+exy+fx^3+gy^3+hx^2y+ixy^2$$

b) der Endabschnitt der Fläche (45) durch eine lineare Funktion erster Ordnung definiert ist,

$$f(x,y)= \text{cost}+ax+by$$

**Revendications**

1. Turbine (3) pour spiromètre (1) comprenant un corps tubulaire creux (5) contenant un rotor (12) mobile autour d'un axe longitudinal (K) de ce corps tubulaire (5) et deux déflecteurs (11) placés à une ouverture d'admission (7A) et à une ouverture de sortie (7B) dudit corps (5), le rotor (12) comprenant de petites pales (14, 15) se faisant face et disposées entre lesdits déflecteurs (11), les déflecteurs comprenant un corps annulaire (25) et un point d'appui (21) disposé le long d'un axe longitudinal de ce corps annulaire (25) coïncidant avec l'axe longitudinal (K) du corps tubulaire (5), entre ledit point d'appui (21) et ledit corps annulaire (25) sont prévues plusieurs pales (22) qui sont solidaires du point d'appui lui-même et du corps annulaire, lesdites pales (22) délimitant une pluralité de secteurs (30) permettant le passage de l'air dirigé vers le rotor, et ayant une partie terminale servant à l'admission de l'air (35) et une partie terminale servant à l'évacuation de l'air (36), chaque pale (22) a un développement continu de la surface qui est défini par une pluralité de sections adjacentes et consécutives de la surface qui ont une forme incurvée et une forme plate, et sont interconnectées les unes aux autres, **caractérisées en ce que**, à la partie terminale servant à l'évacuation de l'air (36), chaque pale (22) a une section terminale de la surface (45) plate, rectiligne dans toute sa section transversale, ladite section plane étant sur un plan (P) perpendiculaire à l'axe longitudinal (K) dudit corps tubulaire (5) et orthogonal à un plan (Z) contenant ledit axe longitudinal (K), ladite section de surface d'extrémité plane faisant saillie vers la pale adjacente, ladite section de surface d'extrémité (45) étant configurée pour diriger l'air sortant du déflecteur (11) orthogonalement audit plan (Z) contenant l'axe longitudinal (K) du corps tubulaire et orthogonalement sur le rotor (12).

2. Turbine selon la revendication 1, **caractérisée en ce que** les sections adjacentes et consécutives de chaque pale (22) comprennent une section unique définie par une surface incurvée (3).

3. Turbine selon la revendication 1, **caractérisée en ce qu'**il y a quatre (42-45) sections adjacentes et consécutives de la surface de chaque pale (22).

4. Turbine selon la revendication 3, **caractérisée en ce que**, à partir de la partie d'extrémité d'entrée (35) de la pale (22) et jusqu'à la partie d'extrémité d'évacuation (36) de la pale (22), la pale comprend une première section de surface (42) définie par une surface plane, une deuxième section de surface (43) définie par une surface courbe, une troisième section de surface (44) définie par une surface plane, et une quatrième section de surface définissant la section finale de surface (45) reposant sur un plan, la section finale de surface (45) étant inclinée par rapport à la troisième section de surface (44).

5. Turbine selon la revendication 4, **caractérisée en ce que** la première section de la surface (42) forme un angle (β) avec une paroi (26) du corps annulaire (25) compris entre 0,3° et 0,9°, de préférence entre 0,5° et 0,7°.

6. Turbine selon la revendication 4, **caractérisée en ce que** la troisième section de la surface (44) forme un angle (α) par rapport au plan (P) qui contient la section finale de la surface (45) compris entre 37° et 41°, de préférence entre 39° et 40°.

7. Turbine selon la revendication 1, **caractérisée en ce que** la section finale de la surface a une extension limitée le long de la partie d'extrémité d'évacuation (36) de l'air de chaque pale (22).

8. Turbine selon la revendication 4, **caractérisée en ce que** :

a) la première section de la surface (42), la deuxième section de la surface (43) et la troisième section de la surface (44) peuvent être définies au moyen d'une fonction non linéaire du troisième ordre de la coordonnée z en fonction des coordonnées x et y :

$$f(x,y) = cost+ax+by+cx^2+dy^2+exy+fx^3+gy^3+hx^2y+ixy^2$$

b) la section finale de la surface (45) est définie par une fonction linéaire du premier ordre,

$$f(x,y)= cost+ax+by$$

**Fig. 1**

**Fig. 2**

**Fig. 3**

**Fig. 4**

**Fig. 5**

*Fig. 6*

*Fig. 7*

*Fig. 8*

*Fig. 9*

**Fig. 10**

**Fig. 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0369506 A1
- US 2020129091 A1

- CN 210494075 U